# EUROPÄISCHE PATENTSCHRIFT

(11) **EP 0 943 086 B1**
(45) Veröffentlichungstag und Bekanntmachung des Hinweises auf die Patenterteilung: **25.02.2004**
(21) Anmeldenummer: 97952011.1
(22) Anmeldetag: 29.11.1997
(51) Int. Cl.: G01N 21/35

(54) **VERFAHREN SOWIE VORRICHTUNG ZUR BESTIMMUNG DER GASBESCHAFFENHEIT EINER GASMISCHUNG**
METHOD AND DEVICE FOR DETERMINING THE GASEOUS QUALITY OF A GAS MIXTURE
PROCEDE ET DISPOSITIF POUR DETERMINER LA NATURE D'UN MELANGE GAZEUX

(30) Priorität: 04.12.1996 DE 19650302
(43) Veröffentlichungstag der Anmeldung: 22.09.1999
(73) Patentinhaber: RUHRGAS AKTIENGESELLSCHAFT, 45138 Essen (DE)
(72) Erfinder: KORSMEIER, Wilhelm, D-45665 Recklinghausen (DE); WOLF, Dieter, D-46286 Dorsten (DE); HOPPE, Manfred, D-46284 Dorsten (DE)
(74) Vertreter: Harlacher, Mechthild, Dipl.-Ing.
(86) Internationale Anmeldenummer: PCT/EP1997/006662
(87) Internationale Veröffentlichungsnummer: WO 1998/025128

(56) Entgegenhaltungen:
- GB-A- 2 163 251
- GB-A- 2 186 076
- US-A- 4 507 558
- US-A- 5 333 591
- US-A- 5 537 854

## Beschreibung

Die Erfindung betrifft ein Verfahren zur Bestimmung der Methanzahl einer Gasmischung, insbesondere eines gasförmigen Brennstoffes. Ferner betrifft die Erfindung eine Vorrichtung zur Bestimmung der Methanzahl einer Gasmischung, mit einer an eine die Gasmischung transportierende Gasleitung angeschlossenen Sensoranordnung, sowie einer Auswerteeinheit zur Bestimmung der Methanzahl der Gasmischung aus dem von der Sensoranordnung gelieferten Messsignal.

Unter der Gasbeschaffenheit eines gasförmigen Brennstoffes, beispielsweise Erdgas, versteht der Fachmann die Zusammenfassung der für die Verbrennung und Verteilung wichtigsten Gaseigenschaften, d. h. die Gaszusammensetzung, die brenntechnischen Kenndaten und die Methanzahl.

Erdgas ist eine Zusammensetzung aus den Bestandteilen Methan, Ethan, Propan und Butan. Gasbestandteile können ferner noch höhere Kohlenwasserstoffe sowie die Inertgase Kohlendioxid und Stickstoff sein. Die Zusammensetzung von Erdgas kann je nach Lieferquelle sehr unterschiedlich sein.

Von besonderer Bedeutung ist die Kenntnis der Methanzahl beim Betrieb von Gasmotoren, die zunehmend als Antriebsaggregate für stationäre Anlagen, z.B. im Rahmen der Kraft-Wärme-Kopplung oder in Erdgasfahrzeugen an Bedeutung gewinnen.

Eine wesentliche Eigenschaft eines gasförmigen Brennstoffes bei der Verwendung als Motorkraftstoff ist die Klopffestigkeit des Gases. Unter Klopfen versteht man bei Ottomotoren einen irregulären Verbrennungsverlauf, bei dem es nach dem Zündvorgang und während des dann eintretenden Druckanstieges im Verbrennungsraum zu Selbstzündungen des komprimieren, noch unverbrannten Gemisches kommt. Diese örtlich begrenzten Selbstzündungen mit ihren besonders hohen lokalen Druckgradienten können zu starken Motorschäden führen.

Die Methanzahl ist ein Maß für die Klopffestigkeit von gasförmigen Brennstoffen. Sie wird bestimmt, indem das zu unterzeichnende Gas in einem Prüfmotor unter ausgewählten und konstant zu haltenden Randbedingungen mit einem Referenzgas hinsichtlich seiner Klopffestigkeit verglichen wird. Das Referenzgas besteht aus zwei Komponenten, entweder dem klopffesten Methan und dem klopffreudigen Wasserstoff oder aus Methan und Kohlenstoffdioxid. Die Methanzahl gibt den prozentualen Methananteil einer Methan/Wasserstoff-Mischung an, die in einem Prüfmotor unter definierten Randbedingungen dieselbe Klopfstärke aufweist wie das zu untersuchende Gas. Wenn z. B. ein Erdgas eine Methanzahl von 85 besitzt, so heißt dies, dass unter bestimmten motorischen Bedingungen dieses Erdgas die gleiche Klopfstärke zeigt wie ein Gemisch aus 85 % Methan und 15 % Wasserstoff.

Die Methanzahlen der in Europa flächendeckend zur Verfügung stehenden Erdgase liegen im Bereich zwischen 70 und 96 Einheiten. Änderungen der Gaszusammensetzung führen zu Änderungen der Methanzahl und somit zu Änderungen der Verbrennungscharakteristik der Gasmotoren. Kommt es zusätzlich, etwa durch Zumischung von Flüssiggasen wie Propan und Butan zu einem Absinken der Methanzahl, führt dies zu weiteren Problemen beim Betrieb der Gasmotoren. Ganz allgemein lässt sich sagen, das mit geringer werdender Methanzahl die Klopfneigung während des Verbrennungsprozesses zunimmt. Reines Methan, dessen Methanzahl 100 beträgt, ist sehr klopffest. Am anderen Ende der Skala steht der sehr klopffreudige Wasserstoff mit einer mit einer Methanzahl 0. Während im Erdgas vorhandene Anteile an Propan und Butan die Klopffestigkeit herabsetzen, kann es demgegenüber durch die inerten Gasbestandteile Stickstoff und Kohlendioxid zu einer Erhöhung der Klopffestigkeit kommen. Hierdurch sind sogar Methanzahlen von über 100 möglich. Die Methanzahl ist mit der Oktanzahl bei Benzinmotoren vergleichbar und nicht mit dem Methangehalt des Gases zu verwechseln.

Es sind bereits verschiedene Möglichkeiten bekannt, um Schwankungen bei der Gasbeschaffenheit im Erdgas zu erkennen. So lassen sich mit Gaschromatographen alle wesentlichen Komponenten eines Brenngases analysieren Im Wege dieser Analyse sind auch Rückschlüsse auf die brenntechnischen Kenngrößen oder die Methanzahl möglich. Dieses Verfahren ist jedoch mit hohen Kosten und einem großen Zeitaufwand verbunden, ferner ist die verhältnismäßig lange Reaktionszeit bei der Gaschromatographie als nachteilig anzusehen. Aus diesen Gründen kommt dieses Verfahren für regelungstechnische Zwecke, etwa bei der Steuerung eines Gasmotors, nicht in Betracht.

Eine weitere Möglichkeit, Gasbeschaffenheitsänderungen zu detektieren, besteht in der Feststellung des Brennwertes mittels eines Kalorimeters. Da neben dem Brennwert des Gases zumindest eine weitere Größe, z. B. der Wobbe-Index oder die Dichte, bekannt sein muss, um die Änderung der Gasbeschaffenheit exakt beschreiben zu können, entsteht ein verhältnismäßig hoher technischer und kostenmäßiger Aufwand.

Schließlich ist es bekannt, Gasbeschaffenheitsänderungen mittels Wärmeleitfähigkeitssensoren zu erkennen. Diese verwenden auf einer Membran aufgebrachte Dünnfilmwiderstände, die der Messung der Temperatur des Gasgemisches dienen. Diese Messung wird zur Temperaturkompensation herangezogen, zugleich werden die Dünnfilmwiderstände zur Aufheizung der Membran benutzt. Dieses Messverfahren macht sich die Tatsache zunutze, dass die über die Dünnfilmwiderstände abgegebene Wärme von der Gasbeschaffenheit abhängt. Diese Abhängigkeit ist jedoch über die gesamte Breite der vorkommenden Gaszusammensetzungen nicht eindeutig. Das Anwendungsgebiet der Wärmleitfähigkeitssensoren ist beschränkt, weil bei der Interpretation der Ergebnisse zusätzliche Informationen über die Gaszusammensetzung vorliegen müssen. Die Interpretation des Messergebnisses wird beispielsweise erschwert, wenn einem sich in seiner Beschaffenheit ändernden Erdgas zusätzlich noch Flüssiggas und Luft zugemischt wird, wie dies in Gasversorgungsnetzen heute weit verbreitet ist.

Die GB-A-2 186 078 und die EP-A-0616 207 zeigen Vorrichtungen zum Detektieren von Bestandteilen einer Gasmischung, beispielsweise dem Methangehalt in einer Gasmischung. Die Gasmischung wird einer Infrarotstrahlung ausgesetzt und mittels einer Sensoranordnung der von Gasmischung absorbierte Anteil der Infrarotstrahlung gemessen.

Aus der US-A 5 537 854 ist Verfahren und eine Vorrichtung zur Bestimmung der Beschaffenheit von gasförmigen Brennstoffen mittels einer akustischen Messgröße bekannt.In einer Bypassleitung zu einer Haupt-Versorgungsleitung befindet sich eine Messkammer mit einer Anordnung zur Messung der Schallgeschwindigkeit oder einer anderen akustischen Größe. Mittels einer Korrelation wird aus der gemessenen akustischen Größe die Methanzahl bestimmt.

Daher liegt der Erfindung die Aufgabe zugrunde, ein Verfahren zur Bestimmung der Methanzahl einer Gasmischung, insbesondere eines gasförmigen Brennstoffes, zu entwickeln, mit dem sich die Methanzahl besonders einfach, mit geringem Auswerteaufwand und zuverlässig bestimmen läßt. Ferner soll eine geeignete Vorrichtung zur Bestimmung der Methanzahl geschaffen werden.

Zur Lösung wird bei einem Verfahren der eingangs genannten Art vorgeschlagen, dass die Gasmischung einer Infrarotstrahlung ausgesetzt wird, dass mittels einer Sensoranordnung der von der Gasmischung absorbierte Anteil der Infrarotstrahlung gemessen und hieraus die Methanzahl der Gasmischung bestimmt wird, wobei die Gasmischung ausschließlich durch Diffusion in den Bereich der Infrarotstrahlung gelangt.

Auch bei der Zumischung von Flüssiggas und Luft zum Erdgas führt das erfindungsgemäße Verfahren zu sehr guten Messergebnissen. Infolge der Zumischung von Flüssiggas bzw. Luft verringert sich die Methanzahl, was im Zusammenwirken der abnehmenden Absorption infolge des insgesamt kleineren Anteils an Kohlenwasserstoffen im Erdgas und der Erhöhung der Absorption infolge des höheren Absorptionsgrades der Flüssiggase Propan und Butan im Vergleich zum Methan zu einer wiederum guten Korrelation der Absorption zur Methanzahl führt.

Die Absorption eines Gases ist von der Anzahl der pro Volumeneinheit vorhandenen Moleküle abhängig. Daher haben Druck und Temperatur des Gases einen Einfluss auf das Messergebnis. Von Vorteil ist es daher, durch eine Temperaturkompensation sowie eine den vorhandenen Leitungsdruck berücksichtigende Druckkompensation die Temperatur- und Druckabhängigkeit auszugleichen.

Zur Lösung der der Erfindung zugrunde liegenden Aufgabe wird ferner bei einer Vorrichtung der eingangs genannten Art vorgeschlagen, dass sich die Sensoranordnung aus einer Strahlungsquelle für Infrarotstrahlung und einem der Strahlungsquelle zugeordneten Strahlungsdetektor zusammensetzt, dass die Messkammer über einen Stichkanal an die Gasleitung angeschlossen ist, dass der Stichkanal aus einem Zuströmkanal sowie einem Abströmkanal zusammengesetzt ist und dass der Zuströmkanal mit seinem Anfang in den Strömungsquerschnitt der Gasleitung hineinragt.

Durch den sich am Beginn des Zuströmkanals einstellenden Staudruck wird eine Druckdifferenz erzeugt, die das Messgas in Richtung auf die Messkammer fließen lässt. Dort wird das Messgas in den Abströmkanal umgelenkt. Infolge dieser Strömungsführung wird der Transport des Messgases beschleunigt und die Zeit; innerhalb der Gasbeschaffenheitsänderungen von der Sensoranordnung erkannt werden, reduziert.

Vorzugsweise sind Strahlungsquelle und Strahlungsdetektor zueinander ausgerichtet angeordnet, wobei sich zwischen der Strahlungsquelle und dem Strahlungsdetektor die Messkammer mit der zu bestimmenden Gasmischung befindet. Neben der direkten Ausrichtung von Strahlungsquelle und Strahlungsdetektor aufeinander zu ist es auch möglich, die Strahlungsquelle und den Strahlungsdetektor in einem bestimmten Winkel zueinander anzuordnen. Im Falle dieser winkligen Anordnung ist es dann notwendig, im Bereich der Messkammer eine Reflektorfläche zur Umlenkung des Infrarotstrahlers anzuordnen.

Gemäß einer Ausgestaltung der Vorrichtung befindet sich die Messkammer in einem Gehäuse, an dem die Strahlungsquelle und der Strahlungsdetektor einander gegenüberliegend lösbar befestigt sind, vorzugsweise über jeweils eine Verschraubung.

Gemäß einer weiteren Ausgestaltung der Vorrichtung ist das Gehäuse mit einem verschließbaren Kupplungselement zum Anschluss an ein Referenzgas versehen. Das Referenzgas dient dabei zur Kalibrierung der Sensoranordnung. Bei der Kalibrierung wird das Referenzgas, von dem lediglich einige Milliliter benötigt werden, in die vorhandene Gasleitung eingespeist. Auf dieses Weise wird die Sensoranordnung auch hinsichtlich des tatsächlich vorhandenen Gasdrucks geeicht.

Vorzugsweise befindet sich zwischen dem Ende des Stichkanals und der Messkammer ein Gasfilter. Dieses Gasfilter ist vorzugsweise als Metallfritte ausgebildet, die infolge ihrer Porösität größere Partikel und insbesondere Verschmutzungen von der Messkammer fernhält.

Weitere Einzelheiten werden nachfolgend anhand der Zeichnung und unter Verwendung der darin eingetragenen Bezugszeichen erläutert. Auf der Zeichnung zeigen:
- Fig. 1: in einer Schnittdarstellung eine erste Ausführungsform einer Vorrichtung zur Bestimmung der Gasbeschaffenheit einer Gasmischung,
- Fig. 2: in einer Schnittdarstellung eine zweite Ausführungsform einer Vorrichtung zur Bestimmung der Gasbeschaffenheit einer Gasmischung und
- Fig. 3: in einer Schnittdarstellung eine dritte Ausführungsform einer Vorrichtung zur Bestimmung der Gasbeschaffenheit einer Gasmischung.

An eine Gasleitung 1, durch die Erdgas oder mit Flüssiggas und/oder. Luft vermischtes Erdgas strömt, dessen Methanzahl zu bestimmen ist, ist über eine an der Gasleitung 1 angeschweißte Muffe 2 eine Sensoranordnung 3 befestigt. Die Sensoranordnung ist hierzu mit einem Schraubansatz 4 versehen, der in eine Gewinde der Muffe 2 eingeschraubt wird.

Die Gasleitung 1 kann der Gaszuführung zu einem Gasmotor dienen, dessen Regelung in Abhängigkeit von der Beschaffenheit des zugeführten Erdgases und damit von der mittels der Sensoranordnung 3 ermittelten Methanzahl erfolgt.

Von dem Schraubansatz 4 führt ein Stichkanal 5 zu einer in einem Gehäuse 6 angeordneten Meßkammer 7.. Der Stichkanal 5 eben setzt sich aus einem inneren Kanal, der als Zuströmkanal 8 dient, und einem diesen umgebenden äußeren Kanal, der als Abströmkanal 9 dient, zusammen. Der Zuströmkanal 8 mündet innerhalb des Querschnitts der Gasleitung 1, und ist dort mit einer Anströmschräge 10 versehen, so daß sich an dem dort gelegenen Beginn des Zuströmkanals 8 ein Staudruck einstellt, und ein Gasteilstrom über den Zuströmkanal 8 in Richtung auf die Meßkammer 7 transportiert wird. Vor dem Eintritt in die Meßkammer befindet sich ein als poröse Metallfritte 11 ausgebildetes Gasfilter. Noch vor Erreichen der Metallfritte 11 wird der Gasteilstrom umgelenkt, um dann über den Abströmkanal 9 wieder zurück in die Gasleitung 1 zu gelangen.

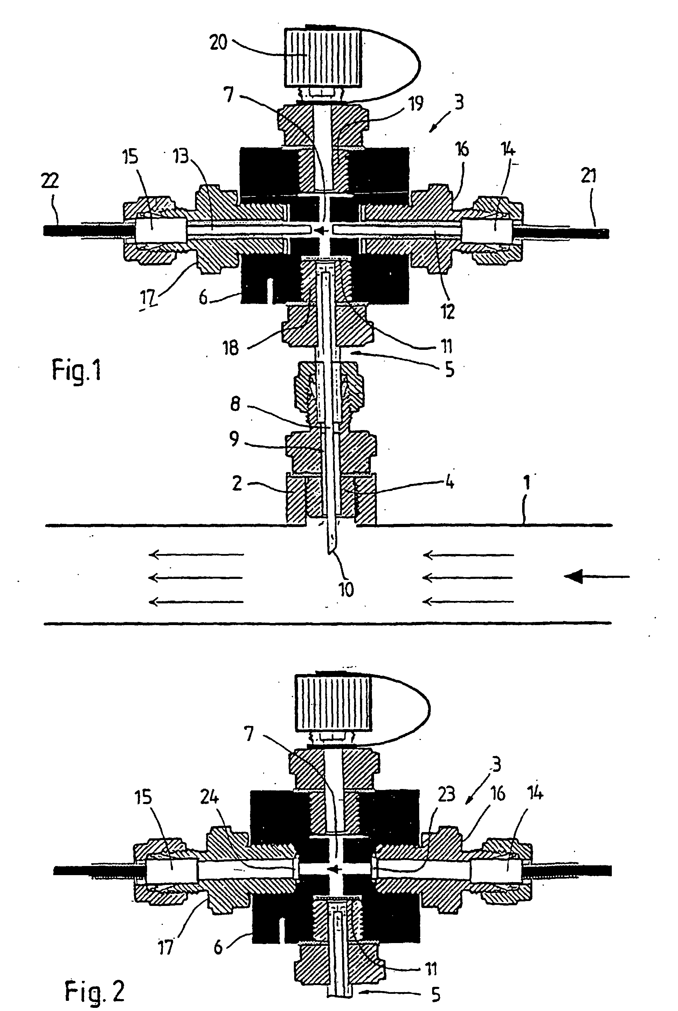

Das Meßgas gelangt ausschließlich infolge von Diffusion durch die Metallfritte 11 hindurch in die dahinter gelegene, nach außen vollständig abgedichtete Meßkammer 7. Die Meßkammer 7 befindet sich zentral innerhalb des Gehäuses 6 der Sensoranordnung 3. Beim Ausführungsbeispiel gemäß Fig. 1 ragen in die Meßkammer 7 zwei Lichtwellenleiter 12, 13, die die Meßkammer 7 gasdicht gegenüber dem Gehäuse 6 abdichten. Der Lichtwellenleiter 12 ist an seinem außenliegenden Ende mit einer Strahlungsquelle 14 für Infrarotstrahlung versehen, der Lichtwellenleiter 13 an seinem äußeren Ende mit einem zugeordneten Strahlungsdetektor 15.

Fig. 1 läßt insbesondere erkennen, daß Strahlungsquelle 14 und Strahlungsdetektor 15 von einander gegenüberliegenden Seiten aus in das Gehäuse 6 eingeschraubt und auf diese Weise zueinander ausgerichtet sind, wobei sich auf der Achse zwischen Strahlungsquelle 14 und Strahlungsdetektor 15 das Zentrum der Meßkammer 7 befindet. Die Infrarot-Strahlungsquelle 14 ist zusammen mit dem zugehörigen Lichtwellenleiter 12 in einem Schraubstutzen 16 untergebracht, der in ein entsprechendes Innengewinde des Gehäuses 6 eingeschraubt ist. Ebenso ist der Strahlungsdetektor 15 mit seinem zugehörigen Lichtwellenleiter 13 in einem ähnlich gestalteten Schraubstutzen 17 untergebracht, der von der anderen Seite her in das Gehäuse 6 einschraubbar ist. Die Lichtwellenleiter 12, 13 ragen jeweils aus den Schraubstutzen 16, 17 heraus, so daß deren aufeinander ausgerichtete Enden auf einem Teil ihrer Länge frei in die Meßkammer 7 für das Meßgas hineinragen.

Auch der Stichkanal 5 ist mittels eines Schraubstutzens 18 mit dem Gehäuse 6 verbunden. Dem Schraubstutzen 18 gegenüberliegend und damit quer zur Meßlinie zwischen Strahlungsquelle 14 und Strahlungsdetektor 15 befindet sich schließlich ein vierter Schraubstutzen 19. Der Schraubstutzen 19 ist an seinem äußeren Ende mittels einer Verschlußkappe 20 verschließbar. Unter der Verschlußkappe 20 befindet sich ein Kupplungselement, über das ein Referenzgas in die Meßkammer 7 eingelassen werden kann. Hierzu befindet sich das Referenzgas in einem von der Sensoranordnung 3 getrennten Vorrat, der über eine flexible Gasleitung an das Kupplungselement angeschlossen werden kann.

Anschlußkabel 21, 22 führen von der Infrarot-Strahlungsquelle 14 sowie dem Infrarot-Strahlungsdetektor 15 zu einer auf der Zeichnung nicht dargestellten Auswerteeinheit. In der Auswerteeinheit erfolgt anhand der von der Sensoranordnung gelieferten Meßsignale die Bestimmung der Methanzahl des in der Meßkammer 7 enthaltenen Meßgases. Bei der Durchführung dieser Messung gibt die Strahlungsquelle 14 eine gerichtete Infrarotstrahlung in Richtung der Meßkammer 7 ab. Das in der Meßkammer 7 enthaltene Gas absorbiert einen Teil dieser Strahlung, so daß der Strahlungsdetektor 15 den nicht absorbierten Anteil der von der Strahlungsquelle 14 ausgesandten Strahlung erfaßt. Der - Anteil der von dem Gas absorbierten Infrarotstrahlung hängt von der Gasbeschaffenheit ab und ist damit zugleich ein Maß für die für die Steuerung des Gasmotors wichtige Methanzahl des über den Stichkanal 5 zugeführten Gases.

Zu der Sensoranordnung 3 gehören außerdem noch ein Temperaturfühler sowie ein Druckfühler. Deren -Signale werden ebenfalls der Auswerteeinheit übermittelt, um das erzielte Meßergebnis hinsichtlich der Temperatur sowie des Drucks zu kompensieren. Dies ist erforderlich, da die Absorptionseigenschaften eines Gases von der Anzahl der pro Volumeneinheit vorhandenen Moleküle und damit wiederum von Temperatur und Druck abhängen.

Bei der Ausführungsform gemäß Fig. 2 fehlen die beiden Lichtwellenleiter Stattdessen wird die Meßkammer 7 durch entlang ihres Randes abgedichtete Quarzglasscheiben 23, 24 begrenzt, die sich jeweils zwischen Strahlungsquelle 14 bzw. Strahlungsdetektor 15 und der Meßkammer 7 befinden. Die Quarzglasscheiben 23, 24 werden mittels der beiden Schraubstutzen 16, 17 in dem Gehäuse 6 fixiert. Die Quarzscheiben 23, 24 lassen die lnfrarotstrahlung nur in jenem Spektrum wirksam werden, in dem sich die Absorptionsbanden von Methan, Ethan, Propan und Butan befinden, so daß die Quarzscheiben 23, 24 zugleich als selektives optisches Filter wirken. Infolge der Abdichtung entlang ihres Randes übernehmen die Quarzscheiben 23, 24 ferner die Gasabdichtung der Meßkammer 7.

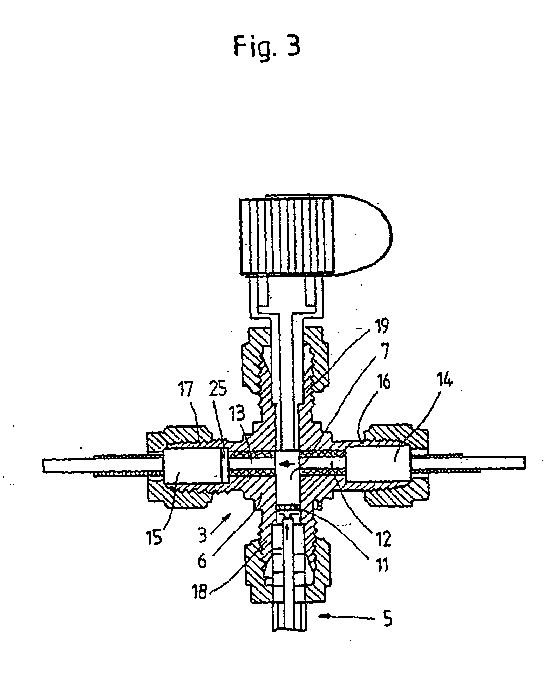

Die Ausführungsform gemäß Fig. 3 zeigt eine-fertigungstechnisch optimierte Ausgestaftung, bei der die Stutzen 16, 17, 18 und 19 einstückig mit dem Gehäuse 6 ausgebildet sind. Um Streuungen und Überlagerungen bei der Detektierung des Infrarotstrahls zu vermeiden, ist bei dem dargestellten Ausführungsbeispiel ein optischer Filter 25 vor dem Strahlungsdetektor 1 5 angeordnet. Die in die Meßkammer 7 hineinragenden Lichtwelienleiter 12 und 13 bewirken auch bei dieser Ausführungsform die Gasabdichtung der Meßkammer 7.

## Patentansprüche

1. Verfahren zur Bestimmung der Methanzahl einer Gasmischung, insbesondere eines gasförmigen Brennstoffes,
**dadurch gekennzeichnet,**
**dass** die Gasmischung einer Infrarotstrahlung ausgesetzt wird, dass mittels einer Sensoranordnung der von der Gasmischung absorbierte Anteil der Infrarotstrahlung gemessen und hieraus die Methanzahl der Gasmischung bestimmt wird, wobei die Gasmischung ausschließlich durch Diffusion in den Bereich der Infrarotstrahlung gelangt.

2. Verfahren nach Anspruch 1,
**dadurch gekennzeichnet, dass** die Bestimmung der Methanzahl in Korrelation zur Größe der gemessenen Absorption erfolgt.

3. Verfahren nach einem der vorangehenden Ansprüche,
**dadurch gekennzeichnet, dass** das von der Sensoranordnung gelieferte Messsignal temperaturkompensiert und/oder druckkompensiert der Bestimmung der Methanzahl zugrunde gelegt wird.

4. Vorrichtung zur Bestimmung der Methanzahl einer Gasmischung, insbesondere eines gasförmigen Brennstoffes, mit einer an eine die Gasmischung transportierende Gasleitung (1) angeschlossenen Sensoranordnung (3), sowie einer Auswerteeinheit zur Bestimmung der Methanzahl der Gasmischung aus dem von der Sensoranordnung (3) gelieferten Messsignal,
**dadurch gekennzeichnet,**
**dass** sich die Sensoranordnung (3) aus einer Strahlungsquelle (14) für Infrarotstrahlung und einem der Strahlungsquelle (14) zugeordneten Strahlungsdetektor (15) zusammensetzt, dass die Messkammer (7) über einen Stichkanal (5) an die Gasleitung (1) angeschlossen ist, dass sich der Stichkanal (5) aus einem Zuströmkanal (8) sowie einem Abströmkanal (9) zusammensetzt und dass der Zuströmkanal (8) mit seinem Anfang in den Strömungsquerschnitt der Gasleitung (1) hineinragt.

5. Vorrichtung nach Anspruch 4, **dadurch gekennzeichnet,**
**dass** Strahlungsquelle (14) und Strahlungsdetektor (15) zueinander ausgerichtet angeordnet sind, und dass sich zwischen der Strahlungsquelle (14) und dem Strahlungsdetektor (15) eine Messkammer (7) mit der zu bestimmenden Gasmischung befindet.

6. Vorrichtung nach Anspruch 5, **dadurch gekennzeichnet,**
**dass** sich die Messkammer (7) in einem Gehäuse (6) befindet, an dem die Strahlungsquelle (14) und der Strahlungsdetektor (15) einander gegenüberliegend lösbar befestigt sind, vorzugsweise über jeweils eine Verschraubung.

7. Vorrichtung nach Anspruch 5 oder 6, **dadurch gekennzeichnet,**
**dass** zwischen der Strahlungsquelle (14) und der Messkammer (7) und/oder zwischen dem Strahlungsdetektor (15) und der Messkammer (7) mindestens ein optischer Filter (25) angeordnet ist.

8. Vorrichtung nach einem der Ansprüche 5 bis 7,
**gekennzeichnet durch** einen die Infrarotstrahlung leitenden gasdicht abdichtend zwischen Strahlungsquelle (14) und Messkammer (7) angeordneten Lichtwellenleiter (12) und/oder einen die Infrarotstrahlung leitenden gasdicht abdichtend zwischen Strahlungsdetektor (15) und Messkammer (7) angeordneten Lichtwellenleiter (13).

9. Vorrichtung nach Anspruch 8,
**dadurch gekennzeichnet, dass** die Strahlungsquelle (14) und/oder der Strahlungsdetektor (15) zusammen mit dem zugehörigen Lichtwellenleiter (12 bzw. 13) in einem in das Gehäuse (6) einschraubbaren Stutzen (16, 17) angeordnet sind.

10. Vorrichtung nach Anspruch 8,
**dadurch gekennzeichnet, dass** der Lichtwellenleiter (12) als eine entlang ihres Randes gasdicht abgedichtete Quarzscheibe (23) zwischen Strahlungsquelle (14) und Messkammer (7) und/oder der Lichtwellenleiter (13) als eine entland ihres Randes gasdicht abgedichtete Quarzscheibe (24) zwischen Strahlungsdetektor (15) und Messkammer (7) ausgebildet ist/sind.

11. Vorrichtung nach Anspruch 10,
**dadurch gekennzeichnet, dass** mindestens eine der Quarzscheiben (23, 24) als optischer Filter dient.

12. Vorrichtung nach einem der Ansprüche 5 bis 11,
**dadurch gekennzeichnet, dass** das Gehäuse (6) mit einem verschließbaren Kupplungselement (Verschlusskappe 20) zum Anschluss an einen Referenzgas-Vorrat versehen ist.

13. Vorrichtung nach einem der Ansprüche 4 bis 12,
**dadurch gekennzeichnet**, und dass sich zwischen dem Ende des Stichkanals (5) und der Messkammer (7) ein Gasfilter (11) befindet.

14. Vorrichtung nach Anspruch 13,
**gekennzeichnet durch** eine Metallfritte als Gasfilter (11).

## Claims

1. Method for determining the methane number of a gas mixture, in particular a gaseous fuel,
**characterised in that**
the gas mixture is subjected to infrared radiation, that the amount of infrared radiation absorbed by the gas mixture is measured using a sensor device and the methane number of the gas mixture determined therefrom, the gas mixture entering the area of the infrared radiation solely by diffusion.

2. Method according to claim 1,
**characterised in that**
the methane number is determined in correlation to the degree of absorption measured.

3. Method according to claim 1 or 2,
**characterised in that**
the measuring signal supplied by the sensor device is temperature-compensated and/or pressure-compensated and then used to determine the methane number.

4. Device for determining the methane number of a gas mixture, in particular a gaseous fuel, with a sensor device (3) connected to a gas line (1) transporting the gas mixture, as well as with an evaluation unit for determining the methane number of the gas mixture from the measuring signal supplied by the sensor device (3),
**characterised in that**
the sensor device (3) is composed of a source of radiation (14) for infrared rays and a radiation sensor (15) assigned to said source of radiation (14), that the measuring chamber (7) is connected to the gas line (1) via a side duct (5), that the side duct (5) is composed of a feed duct (8) and a discharge duct (9) and that the start of the feed duct (8) protrudes into the flow cross-section of the gas line (1)..

5. Device according to claim 4,
**characterised in that**
the source of radiation (14) and the radiation sensor (15) are arranged facing each other and that a measuring chamber (7) with the gas mixture to be determined is located between the source of radiation (14) and the radiation sensor (15).

6. Device according to claim 5,
**characterised in that**
the measuring chamber (7) is located in a housing (6) on which the source of radiation (14) and the radiation sensor (15) are detachably mounted opposite each other, preferably in each case with a screw.

7. Device according to claim 5 or 6,
**characterised in that**
at least one optical filter (25) is arranged between the source of radiation (14) and the measuring chamber (7) and/or between the radiation sensor (15) and the measuring chamber (7).

8. Device according to any one of claims 5 through 7,
**characterised by**
an optical fibre cable (12) arranged between the source of radiation (14) and the measuring chamber (7) sealing gas-tight and carrying the infrared radiation and/or an optical fibre cable (13) arranged between the radiation sensor (15) and the measuring chamber (7) sealing gas-tight and carrying the infrared radiation.

9. Device according to claim 8,
**characterised in that**
the source of radiation (14) and/or the radiation sensor (15) are arranged together with the appertaining optical fibre cable (12 or 13) in a socket (16, 17) which can be screwed into the housing (6).

10. Device according to claim 8,
**characterised in that**
the optical fibre cable (12) is designed as a quartz pane (23, 24) sealing gas-tight along its edge between the source of radiation (14) and the measuring chamber (7) and/or the optical fibre cable (13) is designed as a quartz pane (24) sealing gas-tight along its edge between the radiation sensor (15) and the measuring chamber (7).

11. Device according to claim 10,
**characterised in that**
at least one of the quartz panes (23, 24) serves as an optical filter.

12. Device according to any one of claims 5 through 11,
**characterised in that**
the housing (6) is provided with a sealable coupling element (sealing cap 20) to connect a reference gas reservoir.

13. Device according to any one of claims 4 through 12,
**characterised in that** a gas filter (11) is located between the end of the side duct (5) and the measuring chamber (7).

14. Device according to claim 13,
**characterised by** a metal frit serving as a gas filter (11).

## Revendications

1. Procédé pour déterminer l'indice de méthane d'un mélange gazeux, en particulier d'un combustible gazeux,
**caractérisé par le fait**
**que** le mélange gazeux est exposé à un rayonnement infrarouge, que la part du rayonnement infrarouge absorbée par ledit mélange gazeux est mesurée à l'aide d'une disposition de capteurs et que, de là, l'indice de méthane du mélange gazeux est déterminé, le mélange gazeux accédant à la sphère du rayonnement infrarouge exclusivement par diffusion.

2. Procédé suivant la revendication 1,
**caractérisé par le fait que** l'indice de méthane est déterminé en corrélation avec la grandeur de l'absorption mesurée.

3. Procédé suivant l'une des revendications précédentes,
**caractérisé par le fait que** le signal de mesurage fourni par la disposition de capteurs, compensé par rapport à la température et / ou la pression, est pris comme base pour la détermination de l'indice de méthane.

4. Dispositif pour déterminer l'indice de méthane d'un mélange gazeux, en particulier d'un combustible gazeux, avec une disposition de capteurs (3) raccordée à une conduite de gaz (1) transportant le mélange gazeux, ainsi qu'une unité d'exploitation de données pour déterminer l'indice de méthane du mélange gazeux sur la base du signal de mesurage fourni par la disposition de capteurs (3),
**caractérisé par le fait**
**que** la disposition de capteurs (3) est composée d'une source de rayonnement (14) pour des rayons infrarouge et d'un détecteur de rayonnement (15) attribué à la source de rayonnement (14), que la chambre de mesurage (7) est raccordée à la conduite de gaz (1) par une antenne (5), que l'antenne (5) est composée d'un conduit d'afflux (8) et d'un conduit de reflux (9), et que le conduit d'afflux (8) fait sailli, avec sa partie de départ, dans la section d'écoulement de la conduite de gaz (1).

5. Dispositif suivant la revendication 4,
**caractérisé par le fait que** la source de rayonnement (14) et le détecteur de rayonnement (15) sont alignés l'un par rapport à l'autre et qu'une chambre de mesurage (7) avec le mélange de gaz à déterminer est disposée entre la source de rayonnement (14) et le détecteur de rayonnement (15).

6. Dispositif suivant la revendication 5,
**caractérisé par le fait que** la chambre de mesurage (7) se trouve dans une enveloppe (6), à laquelle la source de rayonnement (14) et le détecteur de rayonnement (15) sont raccordés de manière opposée et de façon détachable, de préférence moyennant un raccord à vis à chaque fois.

7. Dispositif suivant l'une des revendications 5 ou 6,
**caractérisé par le fait qu'** au moins un filtre optique (25) est disposé entre la source de rayonnement (14) et le détecteur de rayonnement (15) et / ou entre le détecteur de rayonnement (15) et la chambre de mesurage (7).

8. Dispositif suivant l'une des revendications 5 à 7,
**caractérisé par** un câble optique (12) guidant le rayonnement infrarouge, disposé de manière étanchant au gaz entre la source de rayonnement (14) et la chambre de mesurage (7) et / ou un câble optique (13) guidant le rayonnement infrarouge, disposé de manière étanchant au gaz entre le détecteur de rayonnement (15) et la chambre de mesurage (7).

9. Dispositif suivant la revendication 8,
**caractérisé par le fait que** la source de rayonnement (14) et / ou le détecteur de rayonnement (15) sont disposés, avec le câble optique correspondant (12 resp. 13), dans une tubulure (16, 17) pouvant être vissée dans l'enveloppe (6).

10. Dispositif suivant la revendication 8,
**caractérisé par le fait que** le câble optique (12) est conçu sous forme de disque en quartz (23), étanche au gaz le long de son bord, entre la source de rayonnement (14) et la chambre de mesurage (7) et / ou le câble optique (13) est conçu sous forme de disque en quartz (24), étanche au gaz le long de son bord, entre le détecteur de rayonnement (15) et la chambre de mesurage (7).

11. Dispositif suivant la revendication 10,
**caractérisé par le fait que** au moins un des disques en quartz (23, 24) sert de filtre optique.

12. Dispositif suivant l'une des revendications 5 à 11,
**caractérisé par le fait que** l'enveloppe (6) est dotée d'un élément d'accouplement pouvant être verrouillé (bouchon de verrouillage 20) pour le raccordement à une réserve de gaz de référence.

13. Dispositif suivant l'une des revendications 4 à 12,
**caractérisé par le fait qu'**un filtre à gaz (11) se trouve entre la fin de l'antenne (5) et la chambre de mesurage (7).

14. Dispositif suivant la revendication 13,
**caractérisé par** une fritte en métal comme filtre à gaz (11).
